# EUROPEAN PATENT SPECIFICATION

(11) **EP 2 275 040 B1**
(45) Date of publication and mention of the grant of the patent: **05.12.2012**
(21) Application number: 10184661.6
(22) Date of filing: 20.10.2001
(51) Int. Cl.: A61B 17/00, A61B 17/34, A61B 17/02

(54) **Wound retraction apparatus**
Wundretraktionsvorrichtung
Appareil d'écartement de lésions

(43) Date of publication of application: 19.01.2011
(62) Divisional of application: 01985170.8
(73) Proprietor: APPLIED MEDICAL RESOURCES CORPORATION, Rancho Santa Margarita, CA 92688 (US)
(72) Inventor: Ewers, Richard C, Fullerton, CA 92833 (US); Brustad, John R, Dana Point, CA 92629 (US); Pingleton, Edward D, San Juan Capistrano, CA 92675 (US); Hilal, Nabil, Laguna Niguel, CA 92677 (US); Adlparvar, Payam, Lake Forest, CA 92630 (US); Taylor, Scott, Mission Viejo, CA 92692 (US); Dulak, Gary R, Newport Beach, CA 92663 (US); Dunn, Michael J, Santa Ana, CA 92706 (US); Morales, Norman, San Jose, CA 95126 (US); Hart, Charles C, Summerville, SC 29483-8949 (US); Bowes, Robert R II, Trabuco Canyon, CA 92679 (US)
(74) Representative: Fitchett, Stuart Paul

(56) References cited:
- EP-A- 1 125 552
- WO-A-00/32116
- WO-A-01/45568
- WO-A-98/48724

## Description

### Field of Invention

This invention relates generally to wound retraction and more specifically to wound retraction in a laparoscopic surgical procedure.

### Discussion of the Related Art

During laparoscopic surgery, it is desirable to inflate the abdominal cavity in order to increase the volume of the working space. This is accomplished with an insufflation gas which must be maintained at a pressure sufficient to inflate the abdomen. Maintaining the pressure of the insufflation gas is difficult when it is also desirable to insert instrumentation through the abdominal wall. If the surgeon is interested in inserting his or her hand in a hand-assisted laparoscopic procedure, the maintenance of insufflation pressure is even more difficult. Currently, several devices exist that accomplish this surgical need although they suffer from drawbacks such as difficult placement and cumbersome use. Thus, it is desirable that the wound be retracted, protected, and fixed while maintaining an insufflation seal. PCT application number WO 01/45568 discloses an adjustable surgical wound retractor comprising a lifter for fine adjustments, European patent application number EP 1,125,552 discloses a surgical retractor for retracting the margins of a wound, and PCT application number WO 00/32116 discloses a surgical device for both retracting and protecting a wound.

### Summary of the Invention

According to the present invention there is provided a surgical wound retractor adapted to dilate a wound, the retractor comprising: a first ring having a diameter greater than a desired diameter of the wound and being adapted for disposition interiorly of the wound; a second ring adapted for disposition exteriorly of the wound; a retraction sleeve having a generally cylindrical configuration with a first end coupled to the first ring and a second end coupled to the second ring; a third ring disposed circumferentially of the retraction sleeve between the first ring and the second ring, the third ring moveable relative to the second ring to adjust a distance between the first ring and the third ring such that the retraction sleeve is provided with the desired radial retraction force, characterized in that the third ring is threadably engaged with the second ring.

Wound retraction in accordance with the present invention allows the surgeon to easily locate a retractor and to provide a solid base for an instrument or hand seal. This retractor removes the tissue pressure from the wrist during hand-assisted laparoscopic surgery. It can also protect the tissue at the wound site, for example, from abrasion, bacteria or other contaminants organs, such as donor kidneys to be removed with minimal risk or damage. The retractor also opens the wound providing greater access to the operative site for instruments, such as the hand of the surgeon.

### Description of the Drawings

FIGS. 1 and 2 are perspective views illustrating the principle of operation of a wound retractor.
FIG. 3 is an axial cross section view of a wound retractor in accordance with the present invention including a threaded second ring and a threaded third ring operable with a sealing cap.

### Description of Preferred Embodiments

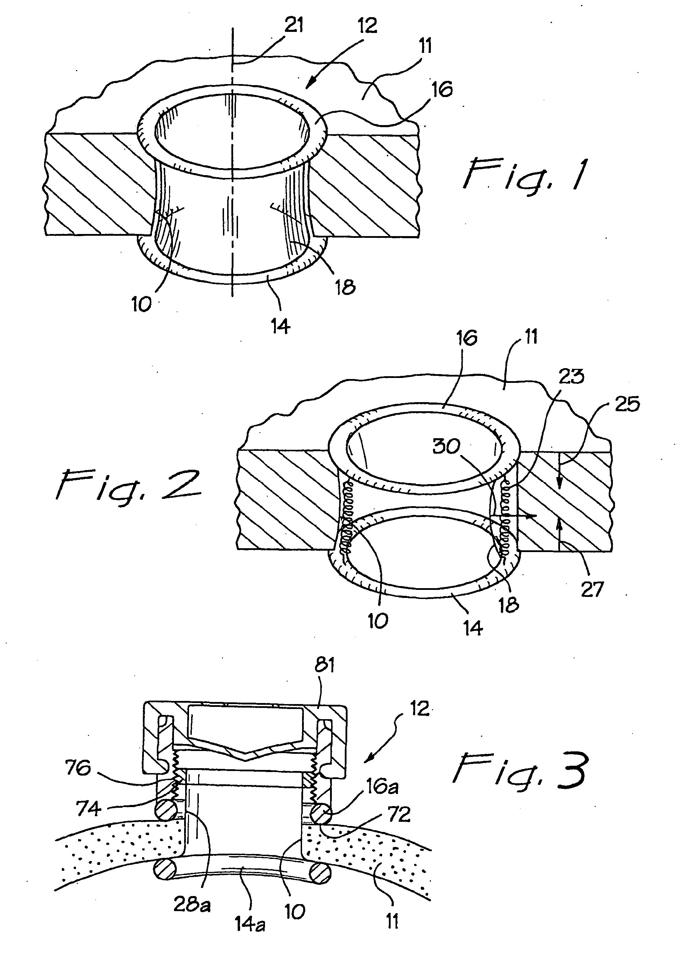

The basic concept of retracting and protecting a wound site is illustrated in the prospective view of Figure 1 wherein a wound 10 is formed in an abdominal wall 11. In this embodiment, a retractor 12 uses two rings 14 and 16 which are fixed to an elastic sheath 18. The sheath 18 has a generally cylindrical configuration and is disposed along an axis 21. The rings 14 and 16 are disposed in respective planes which extend radially of the axis 21.

The sheath 18 has elastomeric properties, but in its natural, unstretched state the two rings 14 and 16 are separate by a natural distance. The lower ring 14 is placed interiorly of the abdominal wall 11 and the upper ring 14 is stretched beyond the natural distance away from the lower ring. Once the elastic sheath 18 has been stretched to a distance greater than the abdominal wall thickness, the upper ring 16 is placed on the surface of the skin.

Since the diameters of the rings 14, 16 are greater than that desired for the wound site 10, they will have sufficient footing to maintain this tension between the two rings 14, 16. This tension is created by the elastic material that has been stretched and retained at a distance greater than the natural distance. It will be appreciated that in many embodiments, the sheath 18 can be formed of a non-elastic sheathing material. In a similar manner, the rings 14 and 16 may be provided with a rigid configuration or alternatively may be formed of an elastromeric material.

Figure 2 shows a simple schematic of the ring dynamics illustrated in Figure 1, with the retractor 12 operatively disposed across the abdominal wall 11. The elastic sheet sheath 18 acts as a circumferential spring 23 around the wound site 10 that evenly distributes the tension between the two rings 14 and 16, as represented by arrows 25 & 27. In addition, the elastic sheeting provides a radial retraction force 30 around the wound to enlarge the wound site 10 in order to facilitate the passage of instruments, such as the hand of the surgeon.

The amount of tension force between the two rings 14 and 16 can be controlled by the elastomeric proportion of the elastic sheath 18. In order to accommodate a larger range of abdominal wall thicknesses, a material with a higher elasticity can be chosen to allow for greater stretch.

Referring to Figure 3, a wound retractor in accordance with the present invention is adapted for opening the wound 10 in the abdominal wall 11 and for maintaining the wound 10 in the open configuration to allow access into the abdominal cavity. The device includes the sheath 28a which lines the wound 10 to prevent or limit the risk of portsite metastasis, and the outer ring 16a which has internal threads 70. The outer ring 16a also includes a sealing surface 72 at its base which is designed to seal against the exterior surface of the abdominal wall 11. The device further comprises the thin film sheath 28a with a ring 74, having external threads 76, which is attached to the proximal end of the sheath 28a, and with the inner ring 14a attached to the distal end of the sheath 28a. The inner ring 14a is designed to be placed inside the wound 10 while the rings 16a and 74 remains external to the wound 10.

Once the inner ring 14a is placed inside the wound 10 and into the peritoneal cavity, the outer ring 16a can be rotated clockwise relative to the inner ring 14a. As the internal thread 70 on the outer ring 16a engage the external threads 76 on the ring 74, the ring 74 is drawn proximally stretching the sheath 28a. This opens the wound 10 and draws both the outer ring 16a and the inner ring 14a toward the abdominal wall 11. A primary seal is created between the inner ring 14a and the abdominal wall 11, while a secondary seal is formed between the surface 72 of the outer ring 16a and the exterior surface of the abdominal wall 11. A sealing cap 81 can then be attached to the proximal end of the outer ring 16a to permit insufflation and otherwise facilitate a laparoscopic procedure. The sealing cap 81 can be removed at any time to allow conversion from laparoscopic surgery to open surgery.

A significant advantage of the invention is that the device enables a surgeon to retract and protectively line an abdominal wall incision, while being able to easily adjust the retractor 12a to accommodate variations from patient to patient in the thickness of the abdominal wall 11. The device effectively seals around the interior and exterior of the wound 10, which allows the sealing cap 81 to be attached to seal the abdominal cavity and enable a laparoscopic procedure to be performed.

Those skilled in the art will envision other modifications within the scope of the present invention as defined by the following claims.

## Claims

1. A surgical wound retractor adapted to dilate a wound, the retractor comprising:
a first ring (14a) having a diameter greater than a desired diameter of the wound and being adapted for disposition interiorly of the wound;
a second ring (74) adapted for disposition exteriorly of the wound;
a retraction sleeve (28a) having a generally cylindrical configuration with a first end coupled to the first ring (14a) and a second end coupled to the second ring (74);
a third ring (16a) disposed circumferentially of the retraction sleeve between the first ring and the second ring, the third ring moveable relative to the second ring to adjust a distance between the first ring and the third ring such that the retraction sleeve is provided with the desired radial retraction force, **characterized in that** the third ring is threadably engaged with the second ring.

2. The retractor of claim 1 further comprising a sealing cap attachable to the third ring.

3. The retractor of claim 2 wherein the sealing cap is removably connectable to the third ring.

4. The retractor of any of claims 2 or 3 wheein the sealing cap is disposed over the third ring.

5. The retractor of any of claims 1-4 wherein the first ring provides a primary seal with the abdominal wall.

6. The retractor of claim 5 wherein a surface of the third ring provides a secondary seal with an exterior surface of the abdominal wall.

## Patentansprüche

1. Chirurgischer Wundspreizer, der so adaptiert wurde, dass er eine Wunde dilatiert, wobei der Spreizer Folgendes umfasst:
einen ersten Ring (14a), der einen Durchmesser hat, der größer als ein gewünschter Durchmesser der Wunde ist, und zur Einstellung innerhalb der Wunde adaptiert wurde,
einen zweiten Ring (74), der zur Einstellung außerhalb der Wunde adaptiert wurde,
eine Retraktionsmanschette (28a), die eine allgemein zylindrische Konfiguration hat, wobei ein erstes Ende am ersten Ring (14a) angekoppelt ist, und ein zweites Ende am zweiten Ring (74) angekoppelt ist,
einen dritten Ring (16a), der umlaufend von der Retraktionsmanschette zwischen dem ersten Ring und dem zweiten Ring angeordnet ist, wobei der dritte Ring beweglich relativ zum zweiten Ring zur Einstellung einer Entfernung zwischen dem ersten Ring und dem dritten Ring ist, sodass die Retraktionsmanschette mit der gewünschten radialen Retraktionskraft versehen ist, **dadurch gekennzeichnet, dass** der dritte Ring gewindemäßig mit dem zweiten Ring in Eingriff steht.

2. Spreizer nach Anspruch 1, der ferner eine Dichtkappe umfasst, die am dritten Ring anfügbar ist.

3. Spreizer nach Anspruch 2, wobei die Dichtkappe entfernbar am dritten Ring anschließbar ist.

4. Spreizer nach einem der Ansprüche 2 oder 3, wobei die Dichtkappe über dem dritten Ring angeordnet ist.

5. Spreizer nach einem der Ansprüche 1 - 4, wobei der erste Ring eine Primärdichtung mit der Bauchdecke darstellt.

6. Wundspreizer nach Anspruch 5, wobei eine Oberfläche des dritten Rings eine Sekundärdichtung mit einer äußeren Oberfläche der Bauchdecke darstellt.

## Revendications

1. Appareil d'écartement de lésions adapté de manière à dilater une lésion, l'appareil d'écartement comprenant :
un premier anneau (14a) ayant un diamètre supérieur à un diamètre désiré de la lésion et étant adapté pour être disposé intérieurement à la lésion ;
un second anneau (74) étant adapté pour être disposé extérieurement à la lésion ;
un manchon rétracteur (28a) ayant une configuration généralement cylindrique avec une première extrémité couplée au premier anneau (14a) et une seconde extrémité couplée au second anneau (74) ;
un troisième anneau (16a) disposé à la circonférence du manchon rétracteur entre le premier anneau et le second anneau, le troisième anneau étant mobile par rapport au second anneau afin d'ajuster une distance entre le premier anneau et le troisième anneau de telle sorte que le manchon rétracteur soit fourni avec la force de rétraction radiale désirée, **caractérisé en ce que** le troisième anneau s'engage par filetage avec le second anneau.

2. Appareil d'écartement selon la revendication 1 comprenant, en outre, un capuchon hermétique attachable au troisième anneau.

3. Appareil d'écartement selon la revendication 2, dans lequel le capuchon hermétique peut être connecté de manière amovible au troisième anneau.

4. Appareil d'écartement selon l'une quelconque des revendications 2 ou 3, dans lequel le capuchon hermétique est disposé sur le troisième anneau.

5. Appareil d'écartement selon l'une quelconque des revendications 1 à 4, dans lequel le premier anneau fournit un joint primaire avec la paroi abdominale.

6. Appareil d'écartement selon la revendication 5, dans lequel une surface du troisième anneau fournit un joint secondaire avec une surface extérieure de la paroi abdominale.
